# EUROPEAN PATENT SPECIFICATION

(11) **EP 2 700 712 B1**
(45) Date of publication and mention of the grant of the patent: **28.12.2016**
(21) Application number: 12773590.0
(22) Date of filing: 28.03.2012
(51) Int. Cl.: C12N 15/09, C12M 1/00, G01N 27/447

(54) **METHOD FOR ELECTROPHORESING NUCLEIC ACIDS, METHOD FOR CONCENTRATING AND PURIFYING NUCLEIC ACIDS, CARTRIDGE FOR NUCLEIC ACID ELECTROPHORESIS, AND METHOD FOR PRODUCING CARTRIDGE FOR NUCLEIC ACID ELECTROPHORESIS**
VERFAHREN ZUR ELEKTROPHORESE VON NUKLEINSÄUREN, VERFAHREN ZUR KONZENTRATION UND REINIGUNG VON NUKLEINSÄUREN, KARTUSCHE ZUR ELEKTROPHORESE VON NUKLEINSÄUREN UND VERFAHREN ZUR HERSTELLUNG EINER KARTUSCHE ZUR ELEKTROPHORESE VON NUKLEINSÄUREN
PROCÉDÉ POUR L'ÉLECTROPHORÈSE D'ACIDES NUCLÉIQUES, PROCÉDÉ POUR CONCENTRER ET PURIFIER DES ACIDES NUCLÉIQUES, CARTOUCHE POUR L'ÉLECTROPHORÈSE D'ACIDE NUCLÉIQUE, ET PROCÉDÉ POUR PRODUIRE UNE CARTOUCHE POUR L'ÉLECTROPHORÈSE D'ACIDE NUCLÉIQUE

(30) Priority: 22.04.2011 JP 2011095972; 22.04.2011 JP 2011095974
(43) Date of publication of application: 26.02.2014
(73) Proprietor: Sony Corporation, Tokyo 108-0075 (JP)
(72) Inventor: NAKAMURA, Tomohiko, Tokyo 108-0075 (JP)
(74) Representative: Müller Hoffmann & Partner
(86) International application number: PCT/JP2012/002137
(87) International publication number: WO 2012/144133

(56) References cited:
- JP-A- 2000 146 911
- BATCHELOR-MCAULEY CHRISTOPHER ET AL: "Voltammetric characterization of DNA intercalators across the full pH range: anthraquinone-2,6-disulfonate and anthraquinone-2-sulfonate.", THE JOURNAL OF PHYSICAL CHEMISTRY. B 25 MAR 2010, vol. 114, no. 11, 25 March 2010 (2010-03-25), pages 4094-4100, XP002732989, ISSN: 1520-5207
- STREKOWSKI ET AL: "Noncovalent interactions with DNA: An overview", MUTATION RESEARCH, ELSEVIER, AMSTERDAM, NL, vol. 623, no. 1-2, 7 September 2007 (2007-09-07), pages 3-13, XP022235501, ISSN: 0027-5107, DOI: 10.1016/J.MRFMMM.2007.03.008
- NETO BRENNO A D ET AL: "Recent developments in the chemistry of deoxyribonucleic acid (DNA) intercalators: principles, design, synthesis, applications and trends.", MOLECULES (BASEL, SWITZERLAND) 2009, vol. 14, no. 5, 2009, pages 1725-1746, XP002732990, ISSN: 1420-3049
- ARMITAGE, B. ET AL.: 'Anthraquinone photonucleases: a surprising role for chloride in the sequence-neutral cleavage of DNA and the footprinting of minor groove-bound ligands' PHOTOCHEMISTRY AND PHOTOBIOLOGY vol. 66, no. 2, August 1997, pages 164 - 170, XP055128320
- CASTANO-ALVAREZ, M. ET AL.: 'Electroactive intercalators for DNA analysis on microchip electrophoresis' ELECTROPHORESIS vol. 28, no. 24, December 2007, pages 4679 - 4689, XP055128321
- CHU, B.C. ET AL.: 'Derivatization of unprotected polynucleotides' NUCLEIC ACIDS RESEARCH vol. 11, no. 18, 24 September 1983, pages 6513 - 6529, XP055128325

## Description

### Technical Field

The present disclosure relates to methods of electrophoresing nucleic acids, methods of concentrating and purifying nucleic acids, cartridges for nucleic acid electrophoresis and methods of producing such cartridges. More specifically, the present disclosure relates to cartridges, or the like, which concentrate and purify nucleic acids by electrophoresis in channels.

### Background Art

Nucleic acid amplification reactions such as PCR (Polymerase Chain Reaction) and LAMP (Loop-Mediated Isothermal Amplification) have been applied in various fields in Biotechnology. For example, in the medical field, diagnoses based on nucleotide sequences of DNAs and RNAs have been conducted, and in the agricultural field, DNA tests have been utilized in techniques such as determining genetically modified crops.

Nucleic acid amplification reactions enable detecting nucleic acids in a small amount of sample by amplifying the nucleic acids with high efficiency. However, if the amount of the nucleic acid contained in the sample is very small, the amount may become less than the detection limit. Further, if a concentration of the nucleic acid in the sample is very small, the nucleic acid may become undetectable because a nucleic acid to be amplified may not be included in the sample of a volume that can be introduced into the reaction field. In these cases, concentrating the nucleic acid in the sample before introducing the sample into the reaction field may be effective.

From the past, as methods of concentrating nucleic acids, a method using phenol/chloroform/ethanol; a method using a column, filter, or the like, for adsorbing nucleic acids; a method using magnetic silica beads, and the like are known. For example, Patent Document 1 discloses a method of concentrating nucleic acids using a porous carrier having the nucleic acid-adsorbing capability. In Non-Patent Document 1, a method of concentrating nucleic acids by a capillary electrophoresis is described.
Patent Document 1: Japanese Patent Application Laid-open No. 2005-080555
Non-Patent Document 1: "On-line sample preconcentration in capillary electrophoresis: Fundamentals and applications." Journal of Chromatography A., Vol.1184 (2008)p.504-541

Armitage, B. et al. (Photochemistry and Photobiology, 66:164-170) discloses anthraquinone photonucleases and investigates the surprising role for chloride in the sequence-neutral cleavage of DNA and the footprinting of minor groove-bound ligands.

Batchelor-McAuley, C. et al. (J. Phys. Chem., vol. 114, no. 11, (2010), pages 4094-4100) relates to the voltametric characterization of DNA intercalactors across the full pH range mentioning among others anthraquinone-2,6-disulofonate and anthraquinone-2-sulfonate.

Strekowski L. et al. (Mutation Research, vol. 623, no. 1-2, (2007) pages 3-13) concerns noncovalent interactions with DNA in the form of an overview.

Neto B. et al. (Molecules, vol. 14, no. 5, (2009) pages 1725-1746) concerns the recent developments in the chemistry of the deoxyribunucleic acid (DNA) intercalactors and deals in particular with principles, design, synthesis, application and trends.

### Summary of Invention

### Problem to be solved by the Invention

The method of the past using phenol/chloroform/ethanol had a necessity of using harmful organic solvents and it took trouble for centrifugation and the like. The method using a column, filter, or the like, for adsorbing nucleic acids had a problem that the column, filter, or the like is prone to clogging, and it also had a problem in terms of ease of operation.

Accordingly, a main object of the present disclosure is to provide a method of electrophoresing nucleic acids, which is capable of highly efficiently concentrating and purifying nucleic acids in a short time by simple operation. This has been achieved by the subject matter of the independent claims.

### Means for solving the Problem

In order to solve the problems described above, the present disclosure provides a method of electrophoresing nucleic acids, which method includes electrophoresing a nucleic acid in which an intercalator having an anionic functional group has been inserted.

In this step of the method of electrophoresing nucleic acids, it is desirable that an acid dissociation constant of the intercalator be more than 0 and 3 or less.

Further, in this method of electrophoresing nucleic acids, it is desirable that the functional group be a sulfo group.

In this method of electrophoresing nucleic acids, the steps of applying a voltage across a channel filled with a buffer so that the nucleic acid introduced at a predetermined position in the channel is electrophoresed; and damming the nucleic acid moving to a positive-electrode end are included.

As used herein, the insertion of an intercalator to a nucleic acid mainly means that an intercalator is inserted between complementary base pair(s) of a double-stranded nucleic acid. Further, in the present disclosure, the term "double-stranded nucleic acid" includes a nucleic acid that is originally made up as a single strand and has become double-stranded partially by self-hybridization.

Further, a pH of the buffer is 5 or less.

Still further, the present disclosure provides a method of concentrating and purifying nucleic acids, which method includes electrophoresing a nucleic acid in which an intercalator having an anionic functional group has been inserted as described above.

Still further, the present disclosure provides a cartridge for nucleic acid electrophoresis, including a channel formed to be capable of introducing a liquid; a damming portion, which dams substances, disposed at a predetermined position of the channel; electrodes disposed at both ends of the channel; an intercalator that can be inserted to a nucleic acid, having an anionic functional group; and a buffer; which intercalator and the buffer are housed between the damming portion and a negative-electrode end.

Furthermore, the present disclosure provides a method of producing said cartridge, which method includes allowing a monomer solution, introduced in a channel formed on a substrate, to gel into a predetermined shape, by photopolymerization, to form the polymer gel formation or disposing a dialysis membrane; and housing an intercalator that can be inserted to a nucleic acid, having an anionic functional group, into the channel.

### Effect of the Invention

According to the present disclosure, there is provided a method of electrophoresing nucleic acids, which is capable of concentrating and recovering nucleic acids with high efficiency in a short time by simple operation.

### Brief Description of Drawings

[Fig. 1] A schematic diagram illustrating a configuration of a cartridge for nucleic acid electrophoresis, and the steps in a method of electrophoresing nucleic acids using the cartridge, according to an embodiment of the present disclosure.
[Fig. 2] A pH-charge curve for explaining an acid dissociation constant according to a first embodiment of the present disclosure.
[Fig. 3] A graph showing changes in fluorescence intensity of a gel interface with the time elapsed from the beginning of the electrophoresis (Example 1: Buffer pH 2.2).
[Fig. 4] A graph showing changes in fluorescence intensity of a gel interface with the time elapsed from the beginning of the electrophoresis (Example 2: Buffer pH 4.0).
[Fig. 5] A view showing a state where a protein (BSA) was electrophoresed (Example 3).
[Fig. 6] A view showing a state where a protein (AGP) was electrophoresed (Example 4).

### Mode(s) for Carrying Out the Invention

Hereinafter, some preferred embodiments for carrying out the present disclosure will be described. It should be noted that the embodiments described below are merely an example of representative embodiments of the present disclosure and should not be interpreted as narrowing the scope of the present disclosure. The description will be given in the following order.
1. A cartridge for nucleic acid electrophoresis, a method of electrophoresing nucleic acids, and a method of producing a cartridge for nucleic acid electrophoresis, according to an embodiment of the present disclosure
   (1) Cartridge for nucleic acid electrophoresis
   (2) Method of electrophoresing nucleic acids
   (3) Method of producing a cartridge for nucleic acid electrophoresis
2. A cartridge for nucleic acid electrophoresis, a method of electrophoresing nucleic acids, and a method of producing a cartridge for nucleic acid electrophoresis, according to an explanatory example representing background art for understanding the invention
   (1) Cartridge for nucleic acid electrophoresis
   (2) Method of electrophoresing nucleic acids
   (3) Method of producing a cartridge for nucleic acid electrophoresis

### 1. A cartridge for nucleic acid electrophoresis, a method of electrophoresing nucleic acids, and a method of producing a cartridge for nucleic acid electrophoresis, according to the embodiment of the present disclosure

### (1) Cartridge for nucleic acid electrophoresis

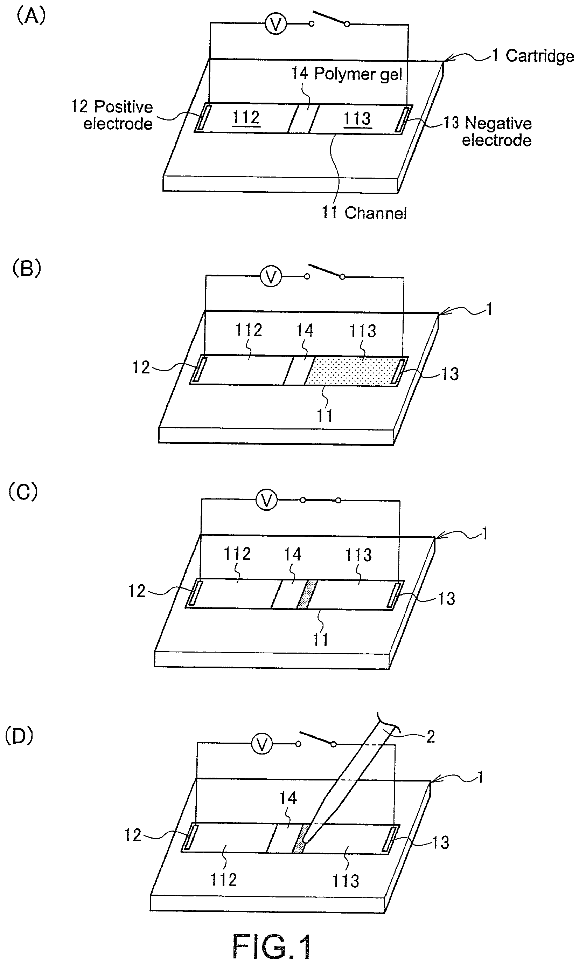

Fig. 1 is a schematic diagram illustrating a configuration of a cartridge for nucleic acid electrophoresis, and the steps in a method of electrophoresing nucleic acids using the cartridge, according to the embodiment of the present disclosure.

A cartridge for nucleic acid electrophoresis in (A) of Fig. 1, denoted by reference numeral 1, has a channel 11 formed on a substrate; and a positive electrode 12 and a negative electrode 13, disposed at both ends of the channel 11. The channel 11 is configured to be capable of introducing a liquid therein. A polymer gel 14 is disposed, between the positive electrode 12 and the negative electrode 13, in the channel 11. Further, a power source V is connected to the positive electrode 12 and the negative electrode 13 such that a voltage can be applied across or removed from the liquid introduced in the channel 11. An intercalator that can be inserted to a nucleic acid; and a buffer are housed in the channel 11.

A material of the substrate may be glass or any of various plastics (polypropylene, polycarbonate, cyclo-olefin polymer, polydimethylsiloxane).

As the positive electrode 12 and the negative electrode 13, desirably, those including gold (Au) or platinum which is sputtered or deposited may be employed.

The polymer gel 14 is an example of the damming portion of the present disclosure. The polymer gel 14 is not especially limited as long as it is capable of damming the nucleic acid in a sample during electrophoresis, as will be described later. In addition, as the damming portion, a porous membrane such as dialysis membrane, reverse osmosis membrane, semipermeable membrane and ion exchange membrane; a porous membrane of a material such as cellulose, polyacrylonitrile, ceramic, zeolite, polysulfone, polyimide and palladium; or the like may be employed. In the following embodiment (the same applies to the explanatory embodiment of the present disclosure), the damming portion of the present disclosure will be described by way of example mainly using the polymer gel 14.

As the polymer gel 14, a polyacrylamide is suitably employed. More desirably, a polyacrylamide containing an anionic functional group may be employed. Still more desirably, a polyacrylamide containing an anionic functional group whose acid dissociation constant (pKa) is from 1 to 5 may be employed. In this embodiment, "acrylamide" is intended to mean an acrylamide or (meth)acrylamide.

Examples of anionic functional groups include, but are not limited to, carboxylic acids such as acetic acid, propionic acid and butyric acid; polybasic acids such as oxalic acid and phthalic acid; hydroxy acids such as citric acid, glycolic acid and lactic acid; unsaturated acids or unsaturated polybasic acids such as acrylic acid and methacrylic acid; amino acids such as glycine; partial esters of phosphoric acid; partial esters of sulfuric acid; phosphoric acid; sulfonic acid; and the like.

Specific examples of the carboxylic acids include aliphatic monocarboxylic acids such as formic acid (pKa: 3.55), acetic acid (pKa: 4.56), propionic acid (pKa: 4.67), butanoic acid (pKa: 4.63), pentenoic acid (pKa: 4.68), hexanoic acid (pKa: 4.63), heptanoic acid (pKa: 4.66), palmitic acid (pKa: 4.64) and stearic acid (pKa: 4.69); aliphatic or aromatic dicarboxylic acids such as succinic acid (pKa₁: 4.00, pKa₂: 5.24), glutaric acid (pKa₁: 4.13, pKa₂: 5.03), adipic acid (pKa₁: 4.26, pKa₂: 5.03), pimelic acid (pKa₁: 4.31, pKa₂: 5.08), suberic acid (pka₁: 4.35, pKa₂: 5.10), azelaic acid (pKa₁: 4.39, pKa₂: 5.12), malic acid (pKa₁: 3.24, pKa₂: 4.71) and terephthalic acid (pKa₁: 3.54, pKa₂: 4.46); unsaturated carboxylic acids such as crotonic acid (pKa: 4.69), acrylic acid (pKa: 4.26) and methacrylic acid (pKa: 4.66); substituted benzoic acids such as anisic acid (pKa: 4.09), *m*-amino benzoic acid (pKa₁: 3.12, pKa₂: 4.74), *m-* or p-chloro benzoic acid (pKa₁: 3.82, pKa₂: 3.99) and hydroxy benzoic acid (pKa₁: 4.08, pKa₂: 9.96); polycarboxylic acids such as citric acid (pKa₁: 2.87, pKa₂: 4.35, pKa₃: 5.69), and derivatives thereof.

As an acrylamide monomer containing an anionic functional group, an acrylamide alkanesulfonic acid may be especially desirable. Examples of the sulfonic acid include styrenesulfonic acid (pKa: -2.8), *m*-anilinesulfonic acid (pKa: 3.74), *p*-anilinesulfonic acid (pKa: 3.23), 2-(meth)acrylamido-2-alkyl (C₁-C₄) propanesulfonic acid, and more specifically, 2-acrylamido-2-methylpropanesulfonic acid (pKa: -1.7), and other sulfonic acids having a polymerizable unsaturated group.

The concentration (wt%) of the anionic functional groups in a polyacrylamide gel may desirably be from 0 to 30%.

The intercalator is not especially limited as long as it is a compound capable of binding to nucleic acids and has an anionic functional group. However, desirably, an acid dissociation constant (pKa) of the intercalator is more than 0 and 3 or less. As used herein, the term "intercalator" refers to a substance to be inserted into the double-strand of the nucleic acid.

Examples of the anionic functional groups contained in the intercalator include a carboxyl group (-COOH group), a mercapto group (-SH group), a phosphoric acid group (-PO₃H), a phosphate group (-PO₄H₂) , a sulfo group (-SO₃H) , a sulfate group (-SO₄H), and the like.

As an anionic functional group contained in the intercalator, a sulfo group may be especially desirable. Example of the intercalators containing a sulfo group include 9,10-anthraquinone-2,6-disulfonic acid, anthraquinone-1-sulfonic acid sodium, anthraquinone-2,7-disulfonic acid disodium, anthraquinone-1,5-disulfonic acid disodium, anthraquinone-2-sulfonic acid sodium, and the like.

A pH of the buffer is not especially limited, but it is desirable to be from 2 to 3, for example. In addition, the pH of the buffer of from 3 to 4 may also be suitable. Furthermore, the pH of the buffer of from 4 to 5 may also be suitable. Examples of the buffers include, but are not limited to, glycine-HCl buffer (pH: 2.0 to 3.0), citrate buffer (pH: 3.0 to 6.0), acetate buffer (pH: 3.0 to 5.5), citrate-phosphate buffer (pH: 2.5 to 7.0), MES (pH: 5.0 to 7.0), Bis-Tris (pH: 5.0 to 7.0), and the like.

### (2) Method of electrophoresing nucleic acids

With reference to (B) to (D) of Fig. 1, the steps in a method of electrophoresing nucleic acids using the cartridge 1 for nucleic acid electrophoresis will be described. The method of electrophoresing nucleic acids mentioned here may be a method of concentrating and purifying nucleic acids.

First, a sample containing a nucleic acid is introduced into an area 113 between the polymer gel 14 and the negative electrode 13 of the channel 11 (see (B) of Fig. 1). By this time, the channel 11 should be filled with a buffer for electrophoresis. Further, the intercalator should be housed as well, in the area 113 between the polymer gel 14 and the negative electrode 13 of the channel 11.

At this time, the intercalator would be inserted into the nucleic acid introduced in the area 113. The step of insertion of the intercalator to the nucleic acid may either be inserting the intercalator to the nucleic acid beforehand and then introducing the nucleic acid into the area 113; or allowing the intercalator housed beforehand in the area 113 to be inserted into the nucleic acid upon introducing the nucleic acid.

As the sample containing a nucleic acid to be used in the method of electrophoresing nucleic acids according to this embodiment, for example, a living body-originated liquid such as throat swab, nasal swab, vaginal swab, blood, plasma, serum, sputum, spinal fluid, urine, sweat and feces may be employed. In addition, as the sample, cell culture liquid, bacterial broth, liquid in food, liquid in soil or the like may also be employed. Because of this, various proteins, sugars and other substances which are unwanted in analyzing nucleic acids (hereinafter, the substances will be referred to as contaminants; and the same applies to the explanatory example) may be contained in the sample liquid. Therefore, in order to analyze a nucleic acid with high efficiency, the nucleic acid is demanded to be separated from the contaminants.

In this respect, as a related art to the present disclosure, there is a method of performing electrophoresis under acid conditions of pH of the buffer and the like (such as pH of from 2 to 4) to separate the nucleic acid and the contaminants, for example. However, in such a method, there is a concern that the electrophoretic velocity of the nucleic acid would be low, and become difficult to concentrate the nucleic acid. In addition, when sialic acid or the like is contained in the contaminants, or when other proteins whose isoelectric point is low are contained, there is a concern that since the isoelectric points of the nucleic acid and the contaminants would be close to each other, it is difficult to separate the nucleic acid and the contaminants even when the electrophoresis is performed.

On the other hand, the intercalator used in this embodiment has an anionic functional group. The pKa of the nucleic acid, where such an intercalator has been inserted, becomes lower, and thus the isoelectric point of the nucleic acid is lowered. As a result, the difference between the isoelectric points of the nucleic acid and the contaminants can be increased, and the electrophoretic velocity of the nucleic acid becomes higher than the electrophoretic velocity of the contaminants. Therefore, the electrophoresis according to this embodiment, which will be described later, enables stably separating the nucleic acid and the contaminants.

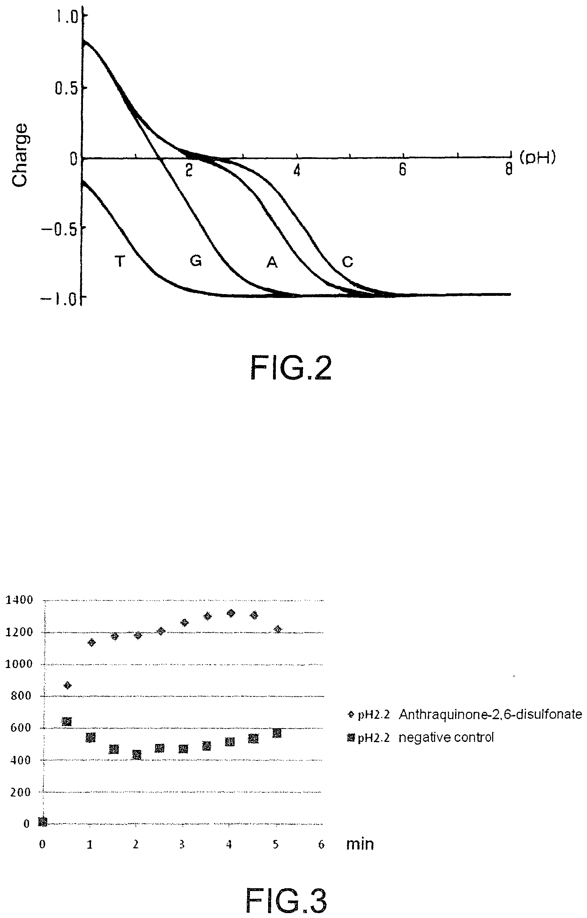

Now, with reference to Fig. 2, an effect of isoelectric points on electrophoresis will be described in detail. Fig. 2 shows a graph of each pH-charge curve corresponding to each base (adenine, guanine, thymine and cytosine, abbreviated in Fig. 2 as A, G, T and C, respectively) that composes a nucleic acid.

As used herein, the term "isoelectric point" refers to a pH at which the charge is 0, as shown in Fig. 2. In any base, a higher pH relative to the isoelectric point gives larger negative charge. Therefore, a substance of lower isoelectric point is able to increase the electrophoretic velocity of the nucleic acid to make the electrophoresis faster. As described above, by inserting the intercalator having an anionic functional group into the nucleic acid, the pH-charge curve of each base shifts to the acid side and lowers the isoelectric point. Regarding each isoelectric point of the corresponding base, for example, the isoelectric point of cytosine (C), which is about from 2.5 to 3, is higher than that of other bases; and therefore, especially with the acid dissociation constant of the intercalator of 3 or less, the electrophoretic velocity of the nucleic acid may be further increased when the nucleic acid is subjected to the insertion of the intercalator.

In addition, by using the buffer having a pH of from 2 to 3, it becomes easier to suppress electrophoresis of the proteins contained in the contaminants, and allows the nucleic acid to be electrophoresed. Therefore, it enables separating the nucleic acid and the contaminants more stably, and purifying the nucleic acid efficiently.

Referring to Fig. 1 again, the method of electrophoresing nucleic acids according to this embodiment, the method of electrophoresing nucleic acids will be described. As described with reference to (B) of Fig. 1, the electrophoresis of the nucleic acid is performed after introducing the nucleic acid into the area 113, by applying a voltage across the positive electrode 12 and the negative electrode 13 so that the voltage is applied to the buffer. The nucleic acid, being negatively charged, is electrophoresed to move within the channel 11 toward the positive electrode 12. At this time, the polymer gel 14 blocks the migration of the nucleic acid, and by damming the nucleic acid, the nucleic acid becomes concentrated at an interface of the polymer gel 14 and in the vicinity thereof (see (C) of Fig. 1). The electrophoresis time may be set as desired depending on the size of the channel 11, and can be, for example, about 10 seconds to 10 minutes. It is to be noted that the term "interface" of the polymer gel means a plane of contact between the polymer gel 14 and the buffer filled in the area 113 side.

Finally, the buffer in the vicinity of the polymer gel 14 in the area 113 is taken up by a micropipette 2 or the like to recover the concentrated nucleic acid (see (D) of Fig. 1).

Thus, according to the method of electrophoresing nucleic acids of this embodiment, the intercalator having an anionic functional group is inserted into the nucleic acid. Further, desirably, the acid dissociation constant of the intercalator is more than 0 and 3 or less. As a result, the negative charge of the nucleic acid becomes strong and the isoelectric point of the nucleic acid becomes lower. In particular, in cases where the intercalator has the functional group with extremely low acid dissociation constant such as sulfo group, the isoelectric point of the nucleic acid in which the intercalator has been inserted becomes significantly lower. Therefore, according to the method of electrophoresing nucleic acids of this embodiment, the electrophoretic velocity of the nucleic acid can be increased, and it can concentrate and purify the nucleic acid in a short time. It should be noted that "inserting an intercalator into a nucleic acid", in the present disclosure, means inserting an intercalator, or in other words, intercalating an intercalator, between complementary base pair(s) of a double-stranded nucleic acid. In addition, in the present disclosure, the term "double-stranded nucleic acid" includes a nucleic acid that is originally made up as a single strand and has become double-stranded partially by self-hybridization.

Further, according to the method of electrophoresing nucleic acids of this embodiment, by lowering the isoelectric point of the nucleic acid as described above, the difference between the isoelectric points of the nucleic acid and the contaminants contained in the sample liquid can be increased. As a result, it can suppress electrophoresis of the contaminants while allowing the nucleic acid to be electrophoresed, and thus it can stably separate the nucleic acid and the contaminants. Therefore, according to the method of electrophoresing nucleic acids of this embodiment, a nucleic acid can be purified with higher purity.

In addition, with the lower pH (such as from 2 to 3) of the buffer to be introduced in the area 113, the electrophoresis of nucleic acids can be performed in a state where the nucleic acid has a negative charge while the negative charge of the contaminants is weakened, which enables separating the nucleic acid and the contaminants with better accuracy.

Further, with the polymer gel 14 containing an anionic functional group, it can prevent the nucleic acid from moving into the polymer gel 14, by an electrical repulsion force between the negatively-charged nucleic acid and the anionic functional group. If the nucleic acid moved into the polymer gel 14, or if it further moved through the polymer gel 14 to reach an area 112 at the positive electrode 12 side, less nucleic acid would be recovered. As the anionic functional group, one with an acid dissociation constant (pKa) of from 1 to 5 may be desirable. Lower pKa and larger negative charge of the functional group provides greater effect of the electrical repulsion force which prevents the nucleic acid from moving into the polymer gel 14.

In addition, for the recovery of the nucleic acid in a greater amount, it is also effective to apply a reverse voltage between the positive electrode 12 and the negative electrode 13 for a short time upon taking up the buffer in the vicinity of the polymer gel 14 in the area 113. The application of the reverse voltage for a short time at the time of the recovery of the nucleic acid makes it possible to move the nucleic acid which exists at the interface of the gel, and the nucleic acid which exists inside the gel near the gel's interface, to the vicinity of the polymer gel 14 in the region 113 so that the nucleic acid can be taken up by the micropipette 2 to be recovered. The application time of the reverse voltage may be set as desired depending on the size of the channel 11, and can be, for example, about 1 to 10 seconds.

### (3) Method of producing a cartridge for nucleic acid electrophoresis

The cartridge for nucleic acid electrophoresis according to this embodiment can be produced by allowing a monomer solution, introduced in the channel 11 formed on the substrate, to gel into a predetermined shape, by photopolymerization, to form the polymer gel 14; and housing the intercalator into the channel 11.

The formation of the channel 11 on the substrate can be conducted, for example, by subjecting a glass substrate layer to wet etching or dry etching, or by subjecting a plastic substrate layer to nanoimprint lithography, injection-molding or cutting. It should be noted that one or more channels 11 can be formed on the cartridge 1.

As the monomer solution, an acrylamide monomer is suitably employed. More desirably, an acrylamide monomer containing an anionic functional group may be employed. Still more desirably, an acrylamide monomer containing an anionic functional group whose acid dissociation constant (pKa) is from 1 to 5 may be employed. As the monomer solution, specifically, an acrylamide alkanesulfonic acid may be especially desirable.

The positive electrode 12 and the negative electrode 13 are prepared desirably by sputtering or deposition of gold (Au) or platinum (Pt). In this respect, cartridges according to a related art include those making use of platinum wires as electrodes. In this case, it would be necessary to arrange the platinum wires in the cartridge for nucleic acid electrophoresis whenever an experiment is performed. Moreover, in view of the high price of the platinum wires, the cartridge has not been provided as a disposable one. On the other hand, as described above, in the cartridge 1 for nucleic acid electrophoresis according to this embodiment, the positive electrode 12 and the negative electrode 13 have been prepared by sputtering or deposition of gold, and as these electrodes have been formed beforehand in the cartridge 1, the cartridge 1 can be handled with ease. Moreover, such a cartridge 1 for nucleic acid electrophoresis can be provided as a disposable one, as compared to the cartridge for nucleic acid electrophoresis according to the related art. Therefore, with the cartridge 1 for nucleic acid electrophoresis, it is possible to avoid contamination of a sample upon its concentration.

### 2. A cartridge for nucleic acid electrophoresis, a method of electrophoresing nucleic acids, and a method of producing a cartridge for nucleic acid electrophoresis, according to an explanatory example representing background art for understanding the invention of the present disclosure

### (1) Cartridge for nucleic acid electrophoresis

Fig. 1, in the same way as one described in the embodiment of the present disclosure, is a schematic diagram illustrating a configuration of a cartridge for nucleic acid electrophoresis, and the steps in a method of electrophoresing nucleic acids using the cartridge, according to the explanatory example of the present disclosure. First, the configuration of the cartridge for nucleic acid electrophoresis according to the explanatory example of the present disclosure will be described with reference to (A) of Fig. 1.

A cartridge for nucleic acid electrophoresis in (A) of Fig. 1, denoted by reference numeral 1, has a channel 11 formed on a substrate; and a positive electrode 12 and a negative electrode 13, disposed at both ends of the channel 11. The channel 11 is configured to be capable of introducing a liquid therein. A polymer gel 14 is disposed, between the positive electrode 12 and the negative electrode 13, in the channel 11. Further, a power source V is connected to the positive electrode 12 and the negative electrode 13 such that a voltage can be applied across or removed from the liquid introduced in the channel 11. A compound which undergoes dehydration condensation reaction with a carboxyl group in contaminants in the sample liquid containing a nucleic acid; and a buffer are housed in the channel 11. Thus, the channel 11, the positive electrode 12, the negative electrode 13 and the polymer gel 14 of the cartridge for nucleic acid electrophoresis according to the explanatory example are substantially the same as those in the embodiment of the present disclosure described above. Therefore, in the cartridge for nucleic acid electrophoresis according to the explanatory example, mainly, the compound which undergoes the dehydration condensation reaction and the buffer will be described.

The compound which undergoes the dehydration condensation reaction is not especially limited as long as it is a compound capable of undergoing dehydration condensation reaction with a carboxyl group of a protein in the contaminants in the nucleic acid-containing sample. The compound desirably has at least a first cationic functional group which undergoes dehydration condensation reaction with a carboxyl group; and a second cationic functional group which exists in an ionic state during nucleic acid electrophoresis. That is, it is desirable that a part of the functional groups of the compound can be electrophoresed in an ionic state, also after the dehydration condensation reaction of the compound and the proteins or the like in the contaminants.

Further, it is desirable that the compound contain one or more amino groups to be amide-linked to the carboxyl group in the proteins of the contaminants.

Specific examples of the compounds include *N-*hydroxysuccinimide (NHS), ethanolamine, ethylenediamine and the like.

Further, it is desirable that a condensing agent for the dehydration condensation reaction be housed in the channel 11. The condensing agent may desirable be a carbodiimide compound. Specific examples of the condensing agents include 1-ethyl-3-(3-dimethylaminopropyl) carbodiimide hydrochloride (EDC), 4-(4,6-dimethoxy-l,3,5-triazin-2-yl)-4-methylmorpholinium chloride (DMT-MM), dicyclohexylcarbodiimide (DCC), diisopylcarbodiimide (DIC) and the like.

In the channel 11 of the cartridge for nucleic acid electrophoresis, it is desirable to house a buffer in addition. A pH of the buffer is not especially limited, but it is desirable to be from 3 to 4. In addition, the pH of the buffer of from 4 to 5 may also be suitable. Furthermore, the pH of the buffer of from 5 to 6 may also be suitable. Still further, the pH of the buffer of from 6 to 7 may also be suitable. Still further, the pH of the buffer of from 7 to 8 may also be suitable. In addition, it is desirable that the buffer do not contain carboxyl or amino groups. Examples of the buffers include glycine-HCl buffer (pH: 2.0 to 3.0), citrate buffer (pH: 3.0 to 6.0), acetate buffer (pH: 3.0 to 5.5), citrate-phosphate buffer (pH: 2.5 to 7.0), Tris (pH: 7.2 to 9.0), MES (pH: 3.0 to 7.0), Bis-Tris (pH: 5.0 to 7.0), PBS (pH: 5.0 to 8.0) and the like.

### (2) Method of electrophoresing nucleic acids

Next, the steps in a method of electrophoresing nucleic acids using the cartridge 1 for nucleic acid electrophoresis will be described with reference to (B) to (D) of Fig. 1.

First, a sample containing a nucleic acid is introduced into an area 113 between the polymer gel 14 and the negative electrode 13 of the channel 11 (see (B) of Fig. 1). By this time, the channel 11 should be filled with a buffer for electrophoresis. Further, the compound described above should be housed, as well, in the area 113 between the polymer gel 14 and the negative electrode 13 of the channel 11.

At this time, there are contaminants contained in the sample liquid containing the nucleic acid introduced in the area 113, and a carboxyl group of a protein in the contaminants undergoes dehydration condensation reaction with the compound described above. The step of allowing the sample liquid and the compound to undergo dehydration condensation reaction may either be performed by introducing the sample liquid to the compound housed beforehand in the area 113; or allowing the compound and the sample liquid to react with each other beforehand and then introducing the sample liquid into the area 113.

Thus, by dehydration condensation reaction of the protein of the contaminants contained in the sample liquid and the compound described above, the negative charge of the protein becomes in a weakened state; and the isoelectric point becomes higher. Therefore, according to the method of electrophoresing nucleic acids of the explanatory example, in the same way as the method of electrophoresing nucleic acids of the first embodiment of the present disclosure, the difference between the isoelectric points of the nucleic acid and the contaminants contained in the sample liquid can be increased.

Subsequently, a method of concentrating and purifying nucleic acids as shown in (C) and (D) of Fig. 1 may be made in a manner substantially the same as the method of concentrating and purifying nucleic acids according to the first embodiment of the present disclosure described above.

Thus, according to the method of electrophoresing nucleic acids of the explanatory example, proteins that are contained in the contaminants in the sample liquid containing the nucleic acid have undergone dehydration condensation reaction with the compound described above. As a result, the negative charge of the proteins after the dehydration condensation reaction becomes weak and the isoelectric point of the nucleic acid becomes higher. In particular, in cases where the compound contains a plurality of cationic functional groups, the isoelectric point of the proteins which have undergone the dehydration condensation reaction with the compound becomes significantly higher. Therefore, even when the electrophoresis of the nucleic acid is performed, migration of the contaminants from negative electrode-side to positive electrode-side becomes difficult. In this way, according to the method of electrophoresing nucleic acids of the explanatory example, the difference between the isoelectric points of the nucleic acid and the contaminants contained in the sample liquid can be increased. Thus, by electrophoresing the nucleic acid in the sample, it can stably separate the nucleic acid and the contaminants. Therefore, according to the method of electrophoresing nucleic acids of the explanatory example, a nucleic acid can be purified with higher purity.

In addition, also with the lower pH of the buffer to be introduced in the area 113, the electrophoresis can be performed in a state where the nucleic acid has a negative charge and the contaminants do not have the negative charge, which enables separating the nucleic acid and the contaminants with better accuracy. Therefore, according to the method of electrophoresing nucleic acids of the explanatory example, concentration factor of nucleic acid can be further increased. Further, according to the method of electrophoresing nucleic acids of the explanatory example, the time to be taken for concentrating can be shortened. By using such a method of electrophoresing nucleic acids, a nucleic acid amplification reaction can be performed with good accuracy.

In addition, in cases where a protein in the contaminants has formed an amide linkage by reacting with N-hydroxysuccinimide, other compounds having amine functional group such as ethanolamine can further substitute N-hydroxysuccinimide to bind to the protein. Since the protein and N-hydroxysuccinimide are relatively weakly bound to each other, the above-mentioned other compounds having amine functional group may be selected as desired.

### (3) Method of producing a cartridge for nucleic acid electrophoresis

The cartridge for nucleic acid concentration and purification according to the explanatory example can be produced by allowing a monomer solution, introduced in the channel 11 formed on the substrate, to gel into a predetermined shape, by photopolymerization, to form the polymer gel 14; and housing the compound described above into the channel 11. Other part such as the formation of the channel 11 may be made in a manner substantially the same as the method of producing the cartridge for nucleic acid electrophoresis according to the above-described first embodiment of the present disclosure.

As described above, a description of the embodiment and the explanatory example has been made. The intercalator described in the first embodiment and dehydration condensing material described in the second embodiment may be used in combination in the present disclosure. That is, the electrophoresis may be performed and the nucleic acid may be concentrated by allowing the nucleic acid to be bound with the intercalator, and, by allowing the proteins of the contaminants in the sample solution to be bound with N-hydroxysuccinimide or the like. By using the intercalator and the compound which undergoes the dehydration condensation reaction in combination, it can increase both the removal efficiency of contaminants and the nucleic acid concentration efficiency. Thus, the nucleic acid can be concentrated, and the like, with higher efficiency in a shorter time.

The present disclosure can take the following configurations.
(1) A method of electrophoresing nucleic acids, the method including:
   electrophoresing a nucleic acid in which an intercalator having an anionic functional group has been inserted.
(2) The method according to (1), in which
   an acid dissociation constant of the intercalator is more than 0 and 3 or less.
(3) The method according to (1) or (2), in which the functional group is a sulfo group.
(4) The method according to any one of (1) to (3), further including:
   applying a voltage across a channel filled with a buffer so that the nucleic acid introduced at a predetermined position in the channel is electrophoresed; and
   damming the nucleic acid moving to a positive-electrode end.
(5) The method according to any one of (4), in which a pH of the buffer is 5 or less.
(6) A method of concentrating and purifying nucleic acids, the method including:
   electrophoresing a nucleic acid in which an intercalator having an anionic functional group has been inserted.
(7) A cartridge for nucleic acid electrophoresis, including:
   a channel formed to be capable of introducing a liquid;
   a damming portion, which dams substances, disposed at a predetermined position of the channel;
   electrodes disposed at both ends of the channel;
   an intercalator that can be inserted to a nucleic acid, having an anionic functional group; and
   a buffer;
   the intercalator and the buffer being housed between the damming portion and a negative-electrode end.
(8) A method of producing a cartridge for nucleic acid electrophoresis, the method including:
   allowing a monomer solution, introduced in a channel formed on a substrate, to gel into a predetermined shape, by photopolymerization, to form a polymer gel; and
   housing an intercalator that can be inserted to a nucleic acid, having an anionic functional group, into the channel.
(9) A method of electrophoresing nucleic acids as background art for understanding the invention, the method including:
   mixing a sample containing a nucleic acid, a compound having a functional group which undergoes dehydration condensation reaction with a carboxyl group included in a substance contained in the sample, and a condensing agent of the dehydration condensation reaction; and
   electrophoresing the nucleic acid.
(10) The method according to (9), in which
   the functional group is an amino group.
(11) The method according to (9) or (10), in which
   the compound is ethanolamine or ethylenediamine.
(12) The method according to (3), in which
   the condensing agent includes at least one selected from 1-ethyl-3-(3-dimethylaminopropyl) carbodiimide hydrochloride salt, 4-(4,6-dimethoxy-1,3,5-triazin-2-yl)-4-methylmorpholinium chloride, dicyclohexylcarbodiimide and diisopylcarbodiimide.
(13) The method according to any one of (9) to (12), in which
   the compound has at least
   a first cationic functional group which undergoes dehydration condensation reaction with a carboxyl group and
   a second cationic functional group which exists in an ionic state during the electrophoresis.
(14) The method according to any one of (9) to (13), further including:
   applying a voltage across a channel filled with a buffer so that the nucleic acid introduced at a predetermined position in the channel is electrophoresed; and
   damming the nucleic acid moving to a positive-electrode end.
(15) The method according to (14), in which
   a pH of the buffer is 8 or less.
(16) The method according to any one of (9) to (15), in which
   the sample includes at least one of
   a living body-originated sample containing at least one of throat swab, nasal swab, vaginal swab, blood, plasma, serum, tissue, tissue fragment, sputum, spinal fluid, urine, sweat, and feces; or
   a sample containing at least one of cell culture liquid, bacterial broth, liquid in food, and soil.
(17) A method of concentrating and purifying nucleic acids, the method including:
   mixing a sample containing a nucleic acid, a compound having a functional group which undergoes dehydration condensation reaction with a carboxyl group included in a substance contained in the sample and a condensing agent of the dehydration condensation reaction; and
   electrophoresing the nucleic acid.
(18) A cartridge for nucleic acid electrophoresis, including:
   a channel formed to be capable of introducing a liquid;
   a damming portion, which dams substances, disposed at a predetermined position of the channel;
   electrodes disposed at both ends of the channel;
   a compound having a functional group which undergoes dehydration condensation reaction with a carboxyl group;
   a condensing agent of the dehydration condensation reaction; and
   a buffer;
   the compound, the condensing agent and the buffer being housed between the damming portion and a negative-electrode end.
(19) A method of producing a chip for nucleic acid electrophoresis, the method including:
   allowing a monomer solution, introduced in a channel formed on a substrate, to gel into a predetermined shape, by photopolymerization, to form a polymer gel; and
   housing a compound having a functional group which undergoes dehydration condensation reaction with a carboxyl group, a condensing agent of the dehydration condensation reaction, and a buffer, into the channel.

### Examples

### 1. Production of a cartridge for nucleic acid electrophoresis

On a PMMA substrate, a channel of 5 mm wide, 60 mm long and 2 mm deep was formed. At opposite ends of the channel, a positive electrode and a negative electrode formed of platinum wires (diameter: 0.5 mm, length: 10 mm, product of The Nilaco Corporation) were disposed, respectively. The channel was filled with an acrylamide solution (Solution 2) prepared in accordance with Table 1 and Table 2. Using a confocal laser scanning microscope ("FV1000" by Olympus Corporation), photopolymerization of the acrylamide was conducted to form a polymer gel of 5 mm wide and 3 mm long in the channel. Subsequently, the unpolymerized acrylamide solution was replaced with an electrophoresis buffer (1×TBE).

**[Table 1]**

| | |
|---|---|
| Acrylamide | 1.67 g (16.7%) |
| Bisacrylamide | 0.53 g (5.3%) |
| Acrylamidomethylpropanesulfonic acid (pKa 1) | 0.39 g (3.9%) |
| ×10 TBE (pH 8.3) | 1 mL |
| Distilled water | 6.41 mL |
| Solution 1 | 10 mL |

**[Table 2]**

| | |
|---|---|
| Solution 1 | 1 mL |
| Riboflavin | 0.02 mL |
| TEMED | 0.02 mL |
| Solution 2 | Approx. 1 mL |

### 2. Preparation of sample liquids

A sample liquid (Example 1) to be introduced in the produced cartridge for nucleic acid electrophoresis was prepared in a condition shown in Table 3. As the nucleic acid, a salmon sperm (final concentration: 0.1 mg/mL) was employed. As the buffer, glycine-HCl buffer (pH: 2.2) (final concentration: 50 mM) was used. Further, anthraquinone-2,6-disulfonic acid disodium was used as an intercalator. In addition, SYBR (registered trademark) GREEN I was used as a fluorescent reagent. Besides, as Comparative Example 1, a sample liquid was prepared in the same condition as in Example 1 except that distilled water was used in place of anthraquinone-2,6-disulfonic acid disodium.

Further, as Example 2, a sample liquid was prepared in the same condition as in Example 1 except that acetate buffer (pH: 4.0) (final concentration: 50 mM) was used in place of glycine-HCl buffer (pH: 2.2) (final concentration: 50 mM). In addition, as Comparative Example 2, a sample liquid was prepared in the same condition as in Example 2 except that distilled water was used in place of anthraquinone-2,6-disulfonic acid disodium.

**[Table 3]**

| | |
|---|---|
| Nucleic acid | 10 µL |
| Buffer | 500 µL |
| Anthraquinone-2,6-disulfonic acid disodium | 100 µL |
| SYBR GREEN I | 1 µL |
| Distilled water | 389 µL |
| Total | 1000 µL |

### 3. Electrophoresis of the nucleic acid (1)

Using produced cartridge for nucleic acid electrophoresis, concentration of a nucleic acid was conducted. Between the polymer gel and the negative-electrode side in the channel, 500 µl of the prepared sample liquid (of the four sample liquids prepared in the conditions of Examples 1, 2 and Comparative Examples 1, 2) was introduced. A voltage was applied between the positive electrode and the negative electrode to conduct electrophoresis at 75 V and 1 mA. The moving sample was observed under the confocal laser scanning microscope.

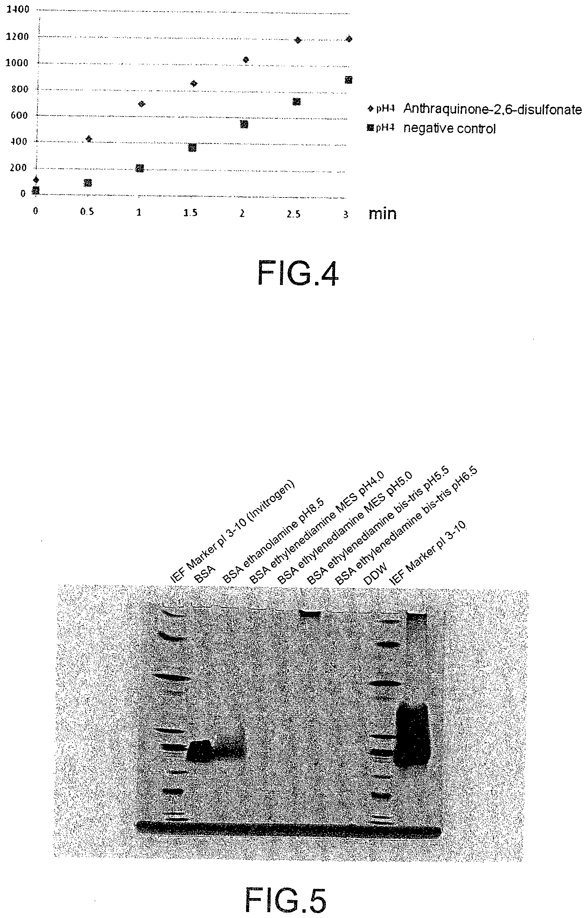

Fig. 3 is a graph showing changes in fluorescence intensity of a gel interface with the time elapsed from the beginning of the electrophoresis using the sample liquids of Example 1 (shown as "pH 2.2 Anthraquinone-2,6-disulfonate" in the figure) and Comparative Example 1 (shown as "pH 2.2 negative control" in the figure). In Fig. 3, the abscissa represents a time (minutes), and the ordinate represents the fluorescence intensity. Fig. 4 is a graph showing changes in fluorescence intensity of a gel interface with the time elapsed from the beginning of the electrophoresis using the sample liquids of Example 2 (shown as "pH 4.0 Anthraquinone-2,6-disulfonate" in the figure) and Comparative Example 2 (shown as "pH 4.0 negative control" in the figure). In Fig. 4, in the same way as in Fig. 3, the abscissa represents a time (minutes), and the ordinate represents the fluorescence intensity.

As shown in Fig. 3, when the buffer with the pH of 2.2 was used, Example 1 using the intercalator was able to realize the concentration rate of approximately twice as high as Comparative Example 1 which did not use the intercalator, after 1 minute from the beginning of the nucleic acid electrophoresis. In addition, as shown in Fig. 4, when the buffer with the pH of 4.0 was used, Example 2 using the intercalator was able to realize the concentration rate of approximately twice as high as Comparative Example 1 which did not use the intercalator, after 1 minute from the beginning of the nucleic acid electrophoresis as well. Thus, it was demonstrated that the insertion of an intercalator having a negative charge into a nucleic acid enables to realize high concentration rate of the nucleic acid in the acid buffer in a short time. Further, it was also demonstrated that by increasing the electrophoretic velocity of the nucleic acid, the difference between the electrophoretic velocities of the nucleic acid and the proteins in the contaminants can be increased, and it can stably separate the nucleic acid and the contaminants.

After finishing the electrophoresis, a reverse voltage (75 V, 1 mA) was applied for 10 seconds between the positive electrode and the negative electrode to liberate the concentrated sample from the gel interface. By bringing a micropipette cartridge, the interior of which was controlled at a negative pressure, into contact with the gel interface, 10 µl of the concentrated sample was recovered.

### 4. Electrophoresis of the nucleic acid (2) (background art for understanding the invention)

In Examples 3 and 4, after subjecting a protein to dehydration condensation reaction, a change in the isoelectric point of the protein was analyzed based on isoelectric focusing. As the protein, Bovine Serum Albumin (BSA) (pI=4.9) 1 mg/mL (Example 3) or α1-acid glycoprotein (AGP) (pI=2.7) 1 mg/mL (Example 4) was employed. As materials which are necessary for the dehydration condensation reaction, NHS 5 mM; EDC 20 mM; and ethanolamine 50 mM or ethylenediamine 50mM; were used. As the buffer, TBE (pH8.5), MES (pH4.0), MES (pH5.0), Bis-Tris (pH6.5) or Bis-Tris (pH5.5) was employed.

Incubation was conducted for 1 hour at room temperature. Then, 10 µl of the samples (Examples 3 and 4) after the dehydration condensation reaction; proteins, as negative controls without undergoing the dehydration condensation reaction, of 1 mg of BSA, 1 mg of AGP (Examples 3 and 4); and IEF Marker pI 3-10 (Invitrogen) were isoelectric-focused.

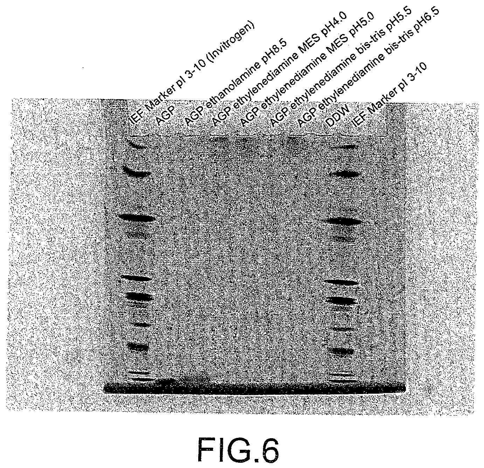

Fig. 5 shows a result of the isoelectric focusing using BSA (Example 3). Fig. 6 shows a result of the isoelectric focusing using AGP (Example 4).

As shown in Figs. 5 and 6, in the negative controls without undergoing the dehydration condensation reaction, the protein bands were observed at a predetermined position. On the other hand, in the samples after the dehydration condensation reaction, there were bands at the predetermined position, with lower density as compared to the proteins without undergoing the dehydration condensation reaction; bands at the position that was moved to the upper side of the gel; and those that were not electrophoresed in the isoelectric focusing gel and could not be observed. From these results, it was demonstrated that the isoelectric points of the proteins were increased by the dehydration condensation reaction. Therefore, it was demonstrated that the difference between the electrophoretic velocities of the nucleic acid and the proteins in the contaminants can be increased, and it can stably separate the nucleic acid and the contaminants.

In addition, when the electrophoresis is performed with the electrophoresis buffer used for usual nucleic acid electrophoresis (pH: 6.0 to 9.0), a sample which has undergone dehydration condensation reaction is electrophoresed to the cathode because of its positive charge. Meanwhile, since a nucleic acid in which the dehydration condensation reaction did not occur has a negative charge, the nucleic acid would be electrophoresed to the anode and thus can be purified. In particular, by setting the pH of the electrophoresis buffer to 6.0 or less, desirably 5.0 or less, and more desirably to more acidic pH, the proteins which have not undergone the dehydration condensation reaction are also able to be separated from the nucleic acid. Thus, the degree of purification of the nucleic acid can be increased.

### Industrial Applicability

A method of electrophoresing nucleic acids according to the present disclosure is capable of concentrating and purifying nucleic acids with high efficiency in a short time by simple operation. Therefore, it can be applied to the concentration treatment of a nucleic acid for nucleic acid amplification reactions such as PCR (Polymerase Chain Reaction) and LAMP (Loop-Mediated Isothermal Amplification), and can be used in order to detect the nucleic acid contained even in a trace amount or at a very low concentration in a sample.

### Description of Reference Numerals

- 1: cartridge
- 2: micropipette
- 11: channel
- 12: positive electrode
- 13: negative electrode
- 14: polymer gel

## Claims

1. A method of electrophoresing nucleic acids, the method comprising:
electrophoresing a nucleic acid in which an intercalator having an anionic functional group has been inserted,
applying a voltage across a channel filled with a buffer so that the nucleic acid introduced at a predetermined position in the channel is electrophoresed; and
damming the nucleic acid moving to a positive-electrode end,
wherein a pH of the buffer is 5 or less.

2. The method according to claim 1, wherein
an acid dissociation constant of the intercalator is more than 0 and 3 or less.

3. The method according to claim 2, wherein
the functional group is a sulfo group.

4. A method of concentrating and purifying nucleic acids, the method comprising:
electrophoresing a nucleic acid in which an intercalator having an anionic functional group has been inserted,
applying a voltage across a channel filled with a buffer so that the nucleic acid introduced at a predetermined position in the channel is electrophoresed; and
damming the nucleic acid moving to a positive-electrode end,
wherein a pH of the buffer is 5 or less

5. A cartridge for nucleic acid electrophoresis, comprising:
a channel formed to be capable of introducing a liquid;
a damming portion, which dams substances, disposed at a predetermined position of the channel;
electrodes disposed at both ends of the channel;
an intercalator that can be inserted to a nucleic acid, having an anionic functional group; and
a buffer;
the intercalator and the buffer being housed between the damming portion and a negative-electrode end.

6. A method of producing the cartridge for nucleic acid electrophoresis of claim 5, the method comprising:
allowing a monomer solution, introduced in a channel formed on a substrate, to gel into a predetermined shape, by photopolymerization, to form a polymer gel; and
housing an intercalator that can be inserted to a nucleic acid, having an anionic functional group, into the channel.

## Patentansprüche

1. Verfahren zur Elektrophorese von Nukleinsäuren, wobei das Verfahren Folgendes umfasst:
Elektrophorese einer Nukleinsäure, in der ein Interkalator mit einer anionischen funktionellen Gruppe inseriert wurde,
Anlegen einer Spannung über einen mit einem Puffer gefüllten Kanal, so dass die an einer vorbestimmten Position im Kanal eingeführte Nukleinsäure einer Elektrophorese unterzogen wird; und
Stauen der sich auf ein Positive-Elektrode-Ende zubewegenden Nukleinsäure,
wobei ein pH des Puffers bei 5 oder darunter liegt.

2. Verfahren nach Anspruch 1, wobei
eine Säuredissoziationskonstante des Interkalators oberhalb von 0 liegt und 3 oder weniger beträgt.

3. Verfahren nach Anspruch 2, wobei
es sich bei der funktionellen Gruppe um eine Sulfo-Gruppe handelt.

4. Verfahren zum Konzentrieren und Aufreinigen von Nukleinsäuren, wobei das Verfahren Folgendes umfasst:
Elektrophorese einer Nukleinsäure, in der ein Interkalator mit einer anionischen funktionellen Gruppe inseriert wurde,
Anlegen einer Spannung über einen mit einem Puffer gefüllten Kanal, so dass die an einer vorbestimmten Position im Kanal eingeführte Nukleinsäure einer Elektrophorese unterzogen wird; und
Stauen der sich auf ein Positive-Elektrode-Ende zubewegenden Nukleinsäure,
wobei ein pH des Puffers bei 5 oder darunter liegt.

5. Kartusche zur Nukleinsäureelektrophorese, umfassend:
einen Kanal, der so ausgebildet ist, dass er zum Einführen einer Flüssigkeit fähig ist;
einen Stauteil, der Substanzen staut, angeordnet an einer vorbestimmten Position des Kanals;
Elektroden, angeordnet an beiden Enden des Kanals;
einen Interkalator, der in eine Nukleinsäure inseriert werden kann, mit einer anionischen funktionellen Gruppe; und
einen Puffer;
wobei der Interkalator und der Puffer zwischen dem Stauteil und einem Negative-Elektrode-Ende untergebracht sind.

6. Verfahren zur Herstellung der Kartusche zur Nukleinsäureelektrophorese gemäß Anspruch 5, wobei das Verfahren Folgendes umfasst:
Gelierenlassen einer Monomerlösung, eingeführt in einem auf einem Substrat ausgebildeten Kanal, in eine vorbestimmte Form durch Photopolymerisation, so dass ein Polymergel gebildet wird; und
Unterbringen eines Interkalators, der in eine Nukleinsäure inseriert werden kann, mit einer anionischen funktionellen Gruppe, in den Kanal.

## Revendications

1. Procédé d'électrophorèse d'acides nucléiques, le procédé comprenant les étapes suivantes :
soumettre à une électrophorèse un acide nucléique dans lequel un agent intercalant ayant un groupe fonctionnel anionique a été inséré,
appliquer une tension à travers un canal rempli avec un tampon de telle sorte que l'acide nucléique introduit à une position prédéterminée dans le canal soit soumis à une électrophorèse ; et
retenir l'acide nucléique se déplaçant vers une extrémité à électrode positive,
dans lequel un pH du tampon est de 5 ou moins.

2. Procédé selon la revendication 1, dans lequel
une constante de dissociation acide de l'agent intercalant est supérieure à 0 et inférieure ou égale à 3.

3. Procédé selon la revendication 2, dans lequel
le groupe fonctionnel est un groupe sulfo.

4. Procédé de concentration et purification d'acides nucléiques, le procédé comprenant les étapes suivantes :
soumettre à une électrophorèse un acide nucléique dans lequel un agent intercalant ayant un groupe fonctionnel anionique a été inséré,
appliquer une tension à travers un canal rempli avec un tampon de telle sorte que l'acide nucléique introduit à une position prédéterminée dans le canal soit soumis à une électrophorèse ; et
retenir l'acide nucléique se déplaçant vers une extrémité à électrode positive,
dans lequel un pH du tampon est de 5 ou moins.

5. Cartouche pour l'électrophorèse d'acides nucléiques, comprenant :
un canal formé pour être en mesure d'introduire un liquide ;
une partie de retenue, qui retient les substances disposées à une position prédéterminée du canal ;
des électrodes disposées aux deux extrémités du canal ;
un agent intercalant qui peut être inséré dans un acide nucléique, ayant un groupe fonctionnel anionique ; et
un tampon ;
l'agent intercalant et le tampon étant accueillis entre la partie de retenue et une extrémité à électrode négative.

6. Procédé de production de la cartouche pour l'électrophorèse d'acides nucléiques de la revendication 5, le procédé comprenant les étapes suivantes :
laisser une solution de monomères, introduite dans un canal formé sur un substrat, gélifier en une forme prédéterminée, par photopolymérisation, pour former un gel polymère ; et
accueillir un agent intercalant qui peut être inséré dans un acide nucléique, ayant un groupe fonctionnel anionique, dans le canal.
